# EUROPÄISCHE PATENTSCHRIFT

(11) **EP 2 308 824 B1**
(45) Veröffentlichungstag und Bekanntmachung des Hinweises auf die Patenterteilung: **03.06.2015**
(21) Anmeldenummer: 10009854.0
(22) Anmeldetag: 17.09.2010
(51) Int. Cl.: C07C 67/08, C07C 67/54, C07C 69/28

(54) **Verfahren zur Herstellung von Polyolestern**
Method for manufacturing polyol esters
Procédé de fabrication d'esters de polyols

(30) Priorität: 08.10.2009 DE 102009048772
(43) Veröffentlichungstag der Anmeldung: 13.04.2011
(73) Patentinhaber: OXEA GmbH, 46147 Oberhausen (DE)
(72) Erfinder: Adamzik, Michael, 46569 Hünxe (DE); Müller, Thomas, 46534 Dinslaken (DE); Schulz, Willy, 46562 Voerde (DE); Schultz, Heyko Jürgen, Dr., 46236 Bottrop (DE)

(56) Entgegenhaltungen:
- DE-A1- 2 226 321
- DE-A1- 10 257 525
- US-A- 5 142 071

## Beschreibung

Die Erfindung betrifft ein Verfahren zur Herstellung von Polyolestern aus linearen oder verzweigten aliphatischen Monocarbonsäuren mit 3 bis 20 Kohlenstoffatomen und Polyolen durch Umsetzung der Ausgangsverbindungen unter partieller Rückführung der aliphatischen Monocarbonsäuren.

Ester mehrwertiger Alkohole, auch Polyolester genannt, finden in großem Umfang und in vielfältiger Weise Anwendung in der Technik, wie zum Beispiel als Weichmacher oder Schmiermittel. Durch die Auswahl geeigneter Ausgangsprodukte lassen sich die physikalischen Stoffeigenschaften, wie zum Beispiel Siedepunkt oder Viskosität gezielt einstellen, und den chemischen Eigenschaften, wie der Hydrolyseresistenz oder der Stabilität gegenüber einem oxidativen Abbau Rechnung tragen. Auch auf die Lösung konkreter anwendungstechnischer Probleme können Polyolester gezielt zugeschnitten werden. Ausführliche Übersichten über den Einsatz von Polyolestern finden sich zum Beispiel in Ullmann's Encyclopedia of Industrial Chemistry, 5. Auflage, 1985, VCH Verlagsgesellschaft, Vol. A1, Seiten 305-319; 1990, Vol. A15, Seiten 438-440 oder in Kirk Othmer, Encyclopedia of Chemical Technology, 3. Auflage, John Wiley & Sons, 1978, Vol. 1, Seiten 778-787; 1981, Vol. 14, Seiten 496-498.

Die Verwendung von Polyolestern als Schmierstoffe besitzt eine große technische Bedeutung und sie werden besonders für solche Anwendungsgebiete eingesetzt, in denen Schmierstoffe auf Mineralölbasis die gesetzten Anforderungen nur unvollständig erfüllen. Polyolester werden insbesondere als Turbinenmotoren- und Instrumentenöle benutzt. Polyolester für Schmiermittelanwendungen basieren häufig auf 1,3-Propandiol, 1,3-Butandiol, 1,4-Butandiol, 1,2-Hexandiol, 1,6-Hexandiol, Neopentylglykol, Trimethylolpropan, Pentaerythrit, 2,2,4-Trimethylpentan-1,3-diol, Glycerin oder 3(4),8(9)-Dihydroxymethyltricyclo[5.2.1.0^{2.6}]decan, auch als TCD-Alkohol DM bezeichnet, als Alkoholkomponente.

Auch als Weichmacher werden Polyolester in erheblichem Umfang verwendet. Weichmacher finden in vielfältiger Weise Anwendung in Kunststoffen, Beschichtungsmitteln, Dichtungsmassen, Kautschuk- und Gummiartikeln. Sie treten mit hochpolymeren thermoplastischen Stoffen, ohne chemisch zu reagieren, vorzugsweise durch ihre Löse- und Quellvermögen in physikalische Wechselwirkung. Hierdurch bildet sich ein homogenes System aus, dessen thermoplastischer Bereich gegenüber den ursprünglichen Polymeren zu niederen Temperaturen verschoben ist, u. a. mit dem Ergebnis, dass seine mechanischen Eigenschaften optimiert, z. B. Formveränderungsvermögen, Elastizität, Festigkeit erhöht werden und die Härte verringert wird.

Um Weichmachern möglichst weite Anwendungsgebiete zu eröffnen, müssen sie eine Reihe von Kriterien erfüllen. Im Idealfall sollten sie geruchlos, farblos, licht-, kälte- und wärmebeständig sein. Überdies erwartet man, dass sie unempfindlich gegenüber Wasser, schwer brennbar und wenig flüchtig sind und die Gesundheit nicht schädigen. Weiterhin soll die Herstellung der Weichmacher einfach sein und, um ökologischen Anforderungen zu genügen, unter Vermeidung von Abfallstoffen, wie nicht weiterverwertbaren Nebenprodukten und Schadstoffe enthaltenden Abwässern, erfolgen.

Eine spezielle Klasse von Polyolestern (sie werden kurz als G-Ester bezeichnet) enthält als Alkoholkomponente Diole bzw. Etherdiole, beispielsweise Ethylenglykol, Diethylenglykol, Triethylenglykol, Tetraethylenglykol, 1,2-Propylenglykol oder höhere Propylenglykole. Ihre Herstellung kann auf unterschiedlichen Wegen erfolgen. Neben der Umsetzung von Alkohol und Säure gegebenenfalls in Gegenwart saurer Katalysatoren werden in der Praxis weitere Prozesse zur Gewinnung von G-Estern angewandt, u. a. die Reaktion von Diol mit Säurehalogenid, die Umesterung eines Carbonsäureesters mit einem Diol und die Addition von Ethylenoxid an Carbonsäuren (Ethoxylierung). In der industriellen Fertigung haben sich lediglich die unmittelbare Umsetzung von Diol und Carbonsäure und die Ethoxylierung von Carbonsäuren als Produktionsverfahren durchgesetzt, wobei der Veresterung von Diol und Säure zumeist der Vorzug gegeben wird. Denn dieses Verfahren kann ohne besonderen Aufwand in konventionellen chemischen Apparaten durchgeführt werden und es liefert chemisch einheitliche Produkte. Demgegenüber erfordert die Ethoxylierung umfangreiche und kostenintensive technische Mittel. Ethylenoxid ist eine sehr aggressive chemische Substanz. Es kann explosionsartig polymerisieren und bildet mit Luft in sehr weiten Mischungsbereichen explosive Gemische. Ethylenoxid reizt die Augen und Atemwege, führt zu Verätzungen, zu Leber- und Nierenschäden und ist karzinogen. Seine Handhabung erfordert daher umfangreiche Sicherheitsmaßnahmen. Überdies ist auf peinliche Sauberkeit von Lagervorrichtungen und Reaktionsapparaten zu achten, um die Bildung unerwünschter Verunreinigungen durch Nebenreaktionen des Ethylenoxids mit Fremdstoffen auszuschließen. Schließlich ist die Reaktion mit Ethylenoxid nicht sehr selektiv, denn sie führt zu Gemischen von Verbindungen unterschiedlicher Kettenlänge.

Die unmittelbare Veresterung von Alkoholen mit Carbonsäuren gehört zu den Grundoperationen der organischen Chemie. Um die Reaktionsgeschwindigkeit zu erhöhen, führt man die Umsetzung üblicherweise in Gegenwart von Katalysatoren durch. Der Einsatz eines der Reaktanten im Überschuss und/oder die Abtrennung des im Verlauf der Reaktion gebildeten Wassers stellt sicher, dass das Gleichgewicht entsprechend dem Massenwirkungsgesetz zur Seite des Reaktionspröduktes, also des Esters, verschoben wird, d. h. hohe Ausbeuten erzielt werden.

Umfassende Angaben zur Herstellung von Estern mehrwertiger Alkohole, darunter auch Ester aus Ethylenglykolen und Fettsäuren und zu den Eigenschaften ausgewählter Vertreter dieser Verbindungsklassen finden sich in Goldsmith, Polyhydric Alcohol Esters of Fatty Acids, Chem. Rev. 33, 257 ff. (1943). Beispielsweise erfolgt die Herstellung von Estern des Diethylenglykols, des Triethylenglykols und von Polyethylenglykolen über Reaktionszeiten von 2,5 bis 8 Stunden bei Temperaturen von 130 bis 230°C. Zur Entfernung des Reaktionswassers verwendet man Kohlendioxid. Als geeignete Katalysatoren für die Veresterung mehrwertiger Alkohole werden anorganische Säuren, saure Salze, organische Sulfonsäuren, Acetylchlorid, Metalle oder amphotere Metalloxide genannt. Die Entfernung des Reaktionswassers erfolgt mit Hilfe eines Schleppmittels, beispielsweise Toluol oder Xylol oder durch Einleiten inerter Gase wie Kohlendioxid oder Stickstoff.

Auf die Gewinnung und die Eigenschaften von Fettsäureestern der Polyethylenglykole geht Johnson (Edit.), Fatty Acids in Industry (1989) Kap. 9, Polyoxyethylene Esters of Fatty Acid ein und gibt eine Reihe präparativer Hinweise. Höhere Diesterkonzentrationen erzielt man durch die Erhöhung des molaren Verhältnisses von Carbonsäure zu Glykol. Geeignete Maßnahmen zur Entfernung des Reaktionswassers sind die Azeotropdestillation in Gegenwart eines mit Wasser nicht mischbaren Lösungsmittels, das Erhitzen unter Durchleiten eines Inertgases oder die Durchführung der Reaktion unter Vakuum in Gegenwart eines Trockenmittels. Verzichtet man auf den Zusatz von Katalysatoren, so sind längere Reaktionszeiten und höhere Reaktionstemperaturen erforderlich.

Beide Reaktionsbedingungen können durch den Einsatz von Katalysatoren gemildert werden. Neben Schwefelsäure sind organische Säuren wie p-Toluolsulfonsäure sowie Kationenaustauscher vom Polystyroltyp die bevorzugten Katalysatoren. Auch die Verwendung von Metallpulvern, wie Zinn oder Eisen, wird beschreiben. Nach der Lehre aus US 2,628,249 lassen sich Farbprobleme bei der Katalyse mit Schwefelsäure oder Sulfonsäuren mildern, wenn in Gegenwart von Aktivkohle gearbeitet wird.

Eine Arbeitsweise, bei der Ester des Diethylen- und Triethylenglykols und der Caprylsäure ohne Katalysatorzusatz hergestellt werden, ist aus US 2,469,446 bekannt. Die Veresterungstemperatur liegt im Bereich von 270 bis 275°C und das Reaktionswasser wird mittels eines Kohlendioxidstromes ausgetrieben.

Bei der Reaktionsführung, bei der man auf die Zugabe eines Katalysators verzichtet, arbeitet man im Allgemeinen mit einem molaren Überschuss an der jeweiligen Carbonsäure, die aufgrund ihrer Acidität auch als Katalysator wirkt.

Für die Abtrennung des bei der Esterbildung aus dem Polyol und der Carbonsäuren gebildeten Reaktionswassers sind verschiedene Verfahren bekannt. Beispielsweise wird das gebildete Reaktionswasser zusammen mit der überschüssigen Carbonsäure aus dem Reaktionsgefäß abdestilliert und in einem nachgeschalteten Phasentrenner geleitet, in dem sich Carbonsäure und Wasser je nach ihren Löslichkeitseigenschaften auftrennen. Gegebenenfalls bildet die eingesetzte Carbonsäure mit Wasser unter den Reaktionsbedingungen auch ein Azeotrop und vermag als Schleppmittel das Reaktionswasser zu entfernen. Anwendung findet auch die Azeotropdestillation in Gegenwart eines zugesetzten mit Wasser nicht mischbaren Lösemittels, das Erhitzen des Reaktionsgemisches unter Durchleiten eines inerten Gases, die Umsetzung der Ausgangsstoffe Polyol und Carbonsäure unter Vakuum oder in Gegenwart eines Trocknungsmittels. Insbesondere die Wasserentfernung durch azeotrope Destillation hat sich für die Einstellung des Gleichgewichts bei der Herstellung von Polyolestern bewährt. Nach der aus DE 199 40 991 A1 bekannten Arbeitsweise wird das mit Wasser nicht mischbare Lösemittel, das als Schleppmittel wirkt und das einen Siedepunkt von weniger als 112°C aufzuweisen hat, dem Reaktionsansatz erst bei Erreichen einer Temperatur von mindesten 140°C zugesetzt.

Der nach Abtrennung des Reaktionswassers und überschüssiger, nicht umgesetzter Ausgangsstoffe, zweckmäßigerweise die im Überschuss zugesetzte Carbonsäure, angefallene Rohester, kann zunächst mit einem alkalischen Reagenz, zum Beispiel mit einer wässrigen Soda- oder Natriumhydroxidlösung behandelt werden, um letzte Reste acider Bestandteile zu entfernen. Nach Wasserwäsche, Behandlung mit Bleicherde und Aktivkohle kann zur Entfernung letzter Spuren von farb- und geruchsgebenden Stoffen Vakuum bei erhöhter Temperatur angelegt werden. Aufarbeitungsverfahren von rohen Polyolestern sind beispielsweise aus US 2,469,446 A1 bekannt. Gegebenenfalls ist die Behandlung mit Bleichmitteln und Aktivkohle mehrmals zu wiederholen, um Endprodukte mit zufriedenstellenden Farbeigenschaften zu gewinnen.

DE 102 57 525 A1 offenbart ein kontinuierliches Verfahren zur Veresterung von alphatischen Fettsäuren mit Polyolen in einer Reaktionskolonne. Das am Kopf der Reaktionskolonne entnommene Gemisch aus Wasser und Leichtsiedern wird aufgetrennt und die Leichtsieder werden wieder in den Veresterungsprozess zurückgeführt. Das über den Sumpf der Reaktionskolonne abgeführte Rohprodukt wird destillativ aufgearbeitet und dabei abgetrennte Komponenten können dem Veresterungsprozess wieder zugeführt werden. US 5,142,071 betrifft die Veresterung von Monoglyceriden mit Fettsäuren, die 6 bis 10 Kohlenstoffatome enthalten. Die nach der Veresterung erhaltenen Triglyceride werden von der überschüssigen Fettsäure durch Destillation oder Wasserdampfbehandlung gereinigt. Die zurückgewonnene Fettsäure kann wieder in den Veresterungsprozess zurückgeführt werden. DE 22 26 321 A1 behandelt die Herstellung von teilveresterten Produkten aus Polyolen mit einer Neopentylpolyol-Struktur durch katalytische Umsetzung mit einer aliphatischen Monocarbonsäure, wobei das gebildete Reaktionswasser und aliphatische Monocarbonsäure so verdampfen, dass die Kondensation und Rückführung des Wassers in das Reaktionsgemisch verhindert wird. Zusätzlich kann dem Reaktionsgemisch aliphatische Monocarbonsäure zugesetzt werden, um im Reaktionsgemisch die Konzentration an der aliphatischen Monocarbonsäure konstant zu halten.

Für die Aufarbeitung des Rohesters schlägt US 5,324,853 A1 vor, die überschüssige Säure mittels Durchleiten von Stickstoff oder Wasserdampf zu entfernen, ein Adsorbens zuzusetzen, mit einer Base restliche organische Säure zu neutralisieren und angefallene Feststoffe abzufiltrieren. Die im Filtrat vorhandenen Restmengen an Säure werden mit dem Durchleiten von Wasserdampf oder Stickstoff bei gleichzeitigem Anlegen eines Unterdrucks entfernt und wieder in die Veresterungsreaktion zurückgeführt. Bei der Vakuumbehandlung angefallene Feststoffe werden in einer abschließenden Feinfiltration entfernt. Gemäß der aus DE 199 40 991 A1 bekannten Arbeitsweise wird der Rohester nach Alkalibehandlung getrocknet, beispielsweise indem man ein inertes Gas durch das Produkt leitet oder Vakuum anlegt und gegebenenfalls noch zusätzlich im Vakuum destilliert. Zur Farbverbesserung von Polyolestern schlägt WO 94/18153 A1 eine nachträgliche Behandlung mit einer wässrigen Wasserstoffperoxidlösung vor.

Wegen der eingangs beschriebenen Qualitätskriterien für Polyolester sind die Verfahrensschritte bei der Veresterungsstufe unter Entfernung des Reaktionswassers und bei der Aufarbeitung des Rohesters sehr wesentliche Prozessmerkmale, denn die Abstimmung dieser Verfahrensschritte beeinflusst in wesentlichem Maße die sensorischen und optischen Eigenschaften der Endprodukte. Insbesondere werden hohe Anforderungen an die Farbeigenschaften, wie geringe Farbzahl und hohe Farbstabilität, der Polyolester gestellt. Die Struktur der Ausgangsstoffe, der mehrwertigen Alkohole und der Säuren, ist dagegen für die mechanischen und thermischen Eigenschaften der mit den Polyolestern weichgestellten Kunststoffmassen maßgebend und beeinflusst die Hydrolyse- und Oxidationsstabilität von Schmiermitteln.

Bei der Herstellung von Polyolestern wird die im Überschuss eingesetzte Carbonsäure, beispielsweise eine aliphatische Monocarbonsäure, im Zuge des Aufarbeitungsverfahrens abgetrennt und wieder in den Veresterungsprozess zurückgeführt. Bei der kontinuierlichen Verfahrensführung erfolgt die Rückführung während des laufenden Prozesses, während bei dem diskontinuierlichen Batch-Prozess die abgetrennte überschüssige aliphatische Monocarbonsäure zunächst gesammelt wird und in dem nächsten Ansatz wieder verwendet wird. Für die Wirtschaftlichkeit des Veresterungsprozesses sind hohe Wiedereinsatzraten der aliphatischen Monocarbonsäure anzustreben. Dem steht jedoch entgegen, dass mit zunehmendem Wiedereinsatz die Säurequalität durch die Bildung und Aufkonzentrierung von Nebenprodukten leidet, so dass im Verlaufe des kontinuierlichen oder des absatzweisen Betriebs die zurückgewonnene aliphatische Monocarbonsäure zumindest teilweise endgültig ausgeschleust und durch Frischsäure ersetzt werden muss. Für ein wirtschaftliches Verfahren ist es jedoch erstrebenswert, die zurückgewonnene aliphatische Monocarbonsäure möglichst häufig in dem kontinuierlichen Veresterungsprozess oder beim absatzweisen Betrieb in den nachfolgenden Produktionsansätzen zu verwenden, ohne dass die Qualität des gewünschten Polyolesters leidet.

Es wurde nun überraschenderweise gefunden, dass sich Polyolester aus Polyolen und linearen oder verzweigten aliphatischen Monocarbonsäuren mit einer ausgezeichneten Farbzahl und Farbstabilität herstellen lassen, wenn die bei der Aufarbeitung des rohen Veresterungsgemisches zurückgewonnene aliphatische Monocarbonsäure nicht vollständig, sondern nur teilweise wieder in die Veresterungsreaktion zurückgeführt wird.

Die Erfindung besteht daher in einem Verfahren zur Herstellung von Polyolestern durch Umsetzung von Polyolen mit linearen oder verzweigten aliphatischen Monocarbonsäuren mit 3 bis 20 Kohlenstoffatomen im Überschuss und anschließende Aufarbeitung des Reaktionsgemisches mittels Wasserdampfbehandlung. Das Verfahren ist dadurch gekennzeichnet, dass man in einem ersten Schnitt die während der Umsetzung abgetrennte aliphatische Monocarbonsäure vollständig oder teilweise aus dem Prozess entfernt, in einem zweiten Schnitt die nach beendeter Umsetzung im Reaktionsgemisch noch vorhandene aliphatische Monocarbonsäure abtrennt und wieder vollständig in die Veresterungsreaktion zurückführt und in einem dritten Schnitt die bei der Wasserdampfbehandlung des Umsetzungsproduktes abgetrennte Restmenge an aliphatischer Monocarbonsäure vollständig aus dem Prozess entfernt, mit der Maßgabe, dass die aliphatische Monocarbonsäure einen niedrigeren Siedepunkt als das eingesetzte Polyol aufweist und sich an die Wasserdampfbehandlung die Trocknung des Polyolesters anschließt.

Durch das gezielte Ausschleusen und Rückführen der während der Umsetzung und anschließenden Aufarbeitungsphase angefallenen aliphatischen Monocarbonsäure lassen sich farbgebenden Komponenten, die während der Veresterungsreaktion entstehen, auf einfache Weise aus dem Prozess entfernen. Es wurde überraschenderweise gefunden, dass sich farbgebende Komponenten in einem ersten Schnitt zusammen mit dem während der Veresterungsreaktion abgetrennten Gemisch aus Reaktionswasser und aliphatischer Monocarbonsäure entfernen lassen. Auch sind in einem dritten Schnitt farbgebende Komponenten in den Restmengen der aliphatischen Monocarbonsäure angesammelt, die im Zuge des Aufarbeitungsverfahrens bei der Wasserdampfbehandlung anfallen. Die im zweiten Schnitt angefallene aliphatische Monocarbonsäure, die im Anschluss an die Veresterungsreaktion aus dem rohen Polyolester abgetrennt wird, ist nur mit geringen Mengen an farbgebenden Komponenten belastet und kann wieder vollständig in den Veresterungsprozess zurückgeführt werden. Bei einer diskontinuierlichen Verfahrensführung bedeutet dies, dass diese Säurefraktion in nachfolgenden Reaktionsansätzen wieder eingesetzt werden kann.

Die erfindungsgmäße Arbeitsweise kann als fraktionierte Destillation der aliphatischen Monocarbonsäure aus dem Reaktionsgemisch und aus dem erhalten Rohester angesehen werden. In einer ersten Fraktion oder in einem ersten Schnitt, den man auch als Vorlauffraktion bezeichnen kann, sammeln sich die farbgebenden Komponenten in dem Gemisch aus aliphatischer Monocarbonsäure und Reaktionswasser an, das während der Veresterungsreaktion abgetrennt wird. In einem zweiten Schnitt, den man auch als Zwischenfraktion bezeichnen kann, wird nahezu reine aliphatische Monocarbonsäure nach beendeter Veresterungsreaktion aus dem Rohester gewonnen, die mit farbgebenden Komponenten nur wenig belastet ist. In einem dritten Schnitt, den man auch als Nachlauffraktion bezeichnen kann, wird wiederum belastete aliphatische Monocarbonsäure im Zuge der Wasserdampfbehandlung erhalten.

Wird das erfindungemäße Verfahren absatzweise durchgeführt, wird die im ersten Schnitt als Vorfraktion und die im dritten Schnitt als Nachlauffraktion erhaltene mit farbgebenden Komponenten belastete aliphatische Monocarbonsäure gesammelt und für die nachfolgenden Produktionsansätze nicht mehr eingesetzt. Bei der kontinuierlichen Prozessführung werden diese Fraktionen kontinuierlich ausgeschleust und wie bei der absatzweisen Fahrweise durch Frischsäure oder durch die wenig belastete Zwischenfraktion aus vorhergehenden Ansätzen ersetzt. Diese abgetrennten Fraktionen können gesammelt in einem separaten Destillationsschritt aufgereinigt und dann wieder in dem Veresterungsprozess eingesetzt werden. Die mit farbgebenden Komponenten wenig belastete Zwischenfraktion oder der zweite Schnitt wird bei der absatzweisen Verfahrensführung für den nächsten Produktionsansatz verwendet oder bei der kontinuierlichen Prozessführung direkt in den Veresterungsreaktor zurückgeführt.

Durch die erfindungsgemäße Maßnahme der fraktionierten Abtrennung und Rückführung der überschüssigen aliphatischen Monocarbonsäure werden die farbgebenden Komponenten in einer geringen Teilmenge der eingesetzten überschüssigen aliphatischen Monocarbonsäure aufkonzentriert während die größere Restmenge der aliphatischen Monocarbonsäure überwiegend frei ist von farbgebenden Komponenten. Diese wenig belastete Zwischenfraktion kann wesentlich häufiger in den Veresterungsprozess zurückgeführt werden als die gesamte belastete abgetrennte überschüssige aliphatische Monocarbonsäure, in der die farbgebenden Komponenten in einer größeren Säuremenge verdünnt vorliegen, ohne dass die Farbqualität des gewünschten Polyolesters zu sehr leidet. Das Entfernen eines kleinen Teils hochbelasteter aliphatischer Monocarbonsäure und die Ergänzung auf den erforderlichen Säureüberschuss durch Zugabe von Frischsäure oder wenig belasteten Zwischenfraktionen aus vorhergehenden Ansätzen nutzt die eingesetzte aliphatische Monocarbonsäure wesentlich ergiebiger als eine Arbeitsweise, bei der ohne Säureergänzung die gesamte, belastete abgetrennte aliphatische Monocarbonsäure für die Veresterungsreaktion wieder verwendet wird. Bei dieser Arbeitsweise muss schon nach wenigen Wiedereinsätzen die gesamte Säuremenge aus dem Prozess entfernt und durch Frischsäure ersetzt werden, um Polyolester mit ausreichender Farbzahl und Farbstabilität zu gewinnen.

Die Reaktion zwischen Polyol und aliphatischer Monocarbonsäure setzt in Abhängigkeit von den Einsatzmaterialien im Bereich von etwa 120 bis 180°C ein und kann auf unterschiedlich ausgestaltete Weise zu Ende geführt werden.

Nach einer Ausgestaltung des erfindungsgemäßen Verfahrens wird zunächst ausgehend von Raumtemperatur auf eine Temperatur bis auf maximal 280°C, vorzugsweise bis auf 250°C erhitzt und bei konstant gehaltener Temperatur der Druck ausgehend von Normaldruck stufenweise erniedrigt, um die Entfernung des Reaktionswassers zu erleichtern. Die Wahl der Druckstufen, ob ein-, zwei- oder mehrstufig, sowie der auf der jeweiligen Stufe einzustellende Druck kann über einen weiten Bereich variiert und den jeweiligen Bedingungen angepasst werden. Beispielsweise kann in einer ersten Stufe der Druck ausgehend von Normaldruck zunächst bis auf 600 hPa erniedrigt werden und anschließend die Reaktion bei einem Druck von 300 hPa zu Ende geführt werden. Bei diesen Druckangaben handelt es sich um Richtwerte, die zweckmäßig eingehalten werden.

Neben der Variation des Drucks kann ebenfalls auch die Temperatur ausgehend von Raumtemperatur während der Veresterungsreaktion ein-, zwei- oder mehrstufig verändert werden, so dass bei konstant eingestelltem Druck die Temperatur von Stufe zu Stufe erhöht wird, üblicherweise bis auf eine maximale Temperatur von 280°C. Es hat sich aber als zweckmäßig erwiesen, bei von Stufe zu Stufe ansteigender Temperatur auf maximal 280°C zu erhitzen und auch den Druck von Stufe zu Stufe zu erniedrigen. Beispielsweise kann die Veresterungsreaktion ausgehend von Raumtemperatur in einer ersten Stufe bei einer Temperatur bis auf 190°C geführt werden. Ebenfalls wird ein verminderter Druck bis auf 600 hPa angelegt, um das Austreiben des Reaktionswassers zu beschleunigen. Nach Erreichen der Temperaturstufe von 190°C wird der Druck nochmals bis auf 300 hPa erniedrigt und die Veresterungsreaktion bei einer Temperatur bis auf 250°C zu Ende geführt. Bei diesen Temperatur- und Druckangaben handelt es sich um Richtwerte, die zweckmäßiger Weise eingehalten werden. Die einzustellenden Temperatur- und Druckbedingungen auf den jeweiligen Stufen, die Zahl der Stufen sowie die jeweilige Temperaturerhöhungs- oder Druckminderungsrate pro Zeiteinheit können über einen weiten Bereich variiert werden und entsprechend den physikalischen Eigenschaften der Ausgangsverbindungen und der Reaktionsprodukte angepasst werden, wobei die Temperatur- und Druckbedingungen der ersten Stufe ausgehend von Normaldruck und Raumtemperatur eingestellt werden. Besonders zweckmäßig hat es sich erwiesen, die Temperatur in zwei Stufen zu erhöhen und den Druck in zwei Stufen zu erniedrigen.

Die untere Grenze des einzustellenden Drucks hängt von den physikalischen Eigenschaften, wie Siedepunkte und Dampfdrücke, der Ausgangsverbindungen sowie der gebildeten Reaktionsprodukte ab und wird auch durch die verfügbaren Anlagenapparate festgelegt. Ausgehend von Normaldruck kann innerhalb dieser Grenzwerte stufenweise mit von Stufe zu Stufe abnehmenden Drücken gearbeitet werden. Die obere Temperaturgrenze, üblicherweise 280°C, ist einzuhalten, um die Bildung von Zersetzungsprodukten, die u. a. farbschädigend wirken, zu vermeiden. Die untere Grenze der Temperaturstufen wird durch die Reaktionsgeschwindigkeit bestimmt, die noch ausreichend hoch sein muss, um die Veresterungsreaktion innerhalb einer vertretbaren Zeit abzuschließen. Innerhalb dieser Grenzwerte kann stufenweise mit von Stufe zu Stufe ansteigenden Temperaturen gearbeitet werden.

Das gebildete Reaktionswasser wird im Laufe der Reaktion zusammen mit der überschüssigen Monocarbonsäure aus dem Reaktionsgefäß abdestilliert und in einen nachgeschalteten Phasentrenner geleitet, in dem sich die Monocarbonsäure und Wasser je nach ihren Löslichkeitseigenschaften auftrennen. Gegebenenfalls bildet die eingesetzte Monocarbonsäure mit Wasser unter den Reaktionsbedingungen auch ein Azeotrop und vermag als Schleppmittel das Reaktionswasser zu entfernen. Aus dem Wasseranfall kann der Reaktionsverlauf verfolgt werden. Das abgeschiedene Wasser wird aus dem Prozess entfernt während die Monocarbonsäure aus dem Phasentrenner wieder in das Reaktionsgefäß zurückfließt. Die Zugabe eines weiteren organischen Lösungsmittels, wie Hexan, 1-Hexen, Cyclohexan, Toluol, Xylol oder Xylolisomerengemische, das die Aufgabe des Azeotropbildners übernimmt, ist nicht ausgeschlossen, jedoch auf wenige Ausnahmefälle beschränkt. Der Azeotropbildner kann bereits zu Beginn der Veresterungsreaktion oder nach Erreichen höherer Temperaturen zugesetzt werden. Wenn die theoretisch zu erwartende Wassermenge angefallen ist oder die Hydroxylzahl, beispielsweise bestimmt nach DIN 53240, unter einen festgelegten Wert gefallen ist, beendet man die Reaktion indem man den Reaktionsansatz abkühlen lässt.

Die nach Beendigung der Umsetzung im Phasentrenner angesammelte aliphatische Monocarbonsäure kann als erster Schnitt oder Vorfraktion bezeichnet werden und ist mit farbgebenden Komponenten belastet. Diese Fraktion wird aus dem Prozess entfernt und in einer separaten Säuredestillation aufgereinigt.

Das nach Beendigung der Umsetzung anfallende Reaktionsgemisch enthält neben dem Polyolester als erwünschtem Reaktionsprodukt noch überschüssige und nicht umgesetzte aliphatische Monocarbonsäure. Sie wird aus dem Rohprodukt, zweckmäßigerweise unter Anlegen eines verminderten Druckes abdestilliert. Die zweckmäßig einzustellenden Destillationsbedingungen ergeben sich aus den physikalischen Eigenschaften des eingesetzten Polyols und der aliphatischen Monocarbonsäure und können durch einfache Vorversuche ermittelt werden. Beispielsweise arbeitet man bei Temperaturen bis 200°C und bei Drücken kleiner als 300 hPa, vorzugsweise kleiner 50 hPa. Die dabei anfallende aliphatische Monocarbonsäure kann man als zweiten Schnitt oder Zwischenfraktion ansehen. Sie ist nur wenig mit farbgebenden Komponenten belastet und kann daher wieder direkt in den Veresterungsprozess zurückgeführt oder als Einsatzmaterial für einen Folgeansatz gesammelt werden.

Die Reaktion von Polyolen und aliphatischen Monocarbonsäuren kann ohne Einsatz eines Katalysators durchgeführt werden. Diese Variante der Umsetzung hat den Vorteil, dass man vermeidet, dem Reaktionsgemisch Fremdstoffe zuzuführen, die zu einer unerwünschten Verunreinigung des Polyolesters führen kann. Allerdings muss man dann im Allgemeinen höhere Reaktionstemperaturen einhalten, weil nur so gewährleistet ist, dass die Umsetzung mit ausreichender, d. h. wirtschaftlich vertretbarer Geschwindigkeit abläuft. Zu beachten ist in diesem Zusammenhang, dass die Steigerung der Temperatur zu einer thermischen Schädigung des Polyolesters führen kann. Daher lässt sich nicht immer die Verwendung eines Katalysators, der die Umsetzung erleichtert und die Reaktionsgeschwindigkeit erhöht, vermeiden. Häufig kann der Katalysator ein Überschuss der aliphatischen Monocarbonsäure sein, die gleichzeitig Reaktionskomponente des Polyols ist, so dass die Reaktion autokatalytisch abläuft. Im Übrigen sind die üblichen Veresterungskatalysatoren zur Beeinflussung der Reaktionsgeschwindigkeit geeignet, wie Schwefelsäure, Ameisensäure, Polyphosphorsäure, Methansulfonsäure oder p-Toluolsulfonsäure und ebenso Kombinationen derartiger Säuren. Ebenfalls verwendet werden können metallhaltige Katalysatoren, wie Titan, Zirkonium oder Zinn enthaltende Katalysatoren, beispielsweise die entsprechenden Alkoholate oder Carboxylate. Auch unter Reaktionsbedingungen feste, im Reaktionssystem unlösliche, katalytisch wirksame Verbindungen wie Alkali- oder Erdalkalihydrogensulfat, beispielsweise Natriumhydrogensulfat können eingesetzt werden. Feste Katalysatoren werden nach Beendigung der Veresterung durch einfache Filtration gegebenenfalls zusammen mit anwesendem Adsorbens aus dem Reaktionsgemisch entfernt. Die Menge des eingesetzten Katalysators kann sich über einen weiten Bereich erstrecken. Es können sowohl 0,001 Gew.-% als auch 5 Gew.-% Katalysator, bezogen auf das Reaktionsgemisch, verwendet werden. Da größere Katalysatormengen jedoch kaum Vorteile ergeben, beträgt die Katalysatorkonzentration üblicherweise 0,001 bis 1,0, vorzugsweise 0,01 bis 0,5 Gew.-%, jeweils bezogen auf das Reaktionsgemisch. Zweckmäßig entscheidet man gegebenenfalls durch Vorversuche für jeden Einzelfall, ob ohne Katalysator bei höherer Temperatur oder mit Katalysator bei niedrigerer Temperatur zu arbeiten ist.

Man lässt das Polyol mit überschüssiger aliphatischer Monocarbonsäure reagieren, üblicherweise ohne Zugabe eines Katalysators, so dass die überschüssige aliphatische Monocarbonsäure selbst als Katalysator wirkt. Auch kann die im Überschuss eingesetzte aliphatische Monocabonsäure als Schleppmittel für das freigesetzte Reaktionswasser dienen und das abgetrennte Wasser/Säuregemisch vermag ebenfalls die farbgebenden Komponenten auszuschleppen. Die Monocarbonsäure weist einen niedrigeren Siedepunkt als das eingesetzte Polyol auf und kann daher auf einfache Weise destillativ aus dem Rohester abgetrennt werden. Die aliphatische Monocarbonsäure wird in einem 10 bis 50%-igen molaren, vorzugsweise in einem 20 bis 40%-igen molaren Überschuss je Mol zu veresternder Hydroxylgruppe des Polyols eingesetzt.

In einer weiteren Ausgestaltung des erfindungsgemäßen Verfahrens wird die Veresterung in Gegenwart eines Adsorbens durchgeführt. Man verwendet dabei poröse, großflächige feste Materalien, die üblicherweise in der chemischen Praxis sowohl im Labor als auch in technischen Anlagen eingesetzt werden. Beispiele für solche Materialien sind oberflächenreiche Polykieselsäuren wie Silicagele (Kiesel-Xerogele), Kieselgel, Kieselguhr, oberflächenreiche Aluminiumoxide und Aluminiumoxidhydrate, mineralische Materialien, wie Tone, Carbonate oder Aktivkohle. Besonders bewährt hat sich Aktivkohle. Im Allgemeinen ist das Adsorbens feinteilig in der Reaktionslösung suspendiert, die durch intensives Rühren oder durch Einleiten eines Inertgases bewegt wird. Dadurch wird ein inniger Kontakt zwischen der flüssigen Phase und dem Adsorbens erreicht. Das Massenverhältnis von flüssiger Phase zu Adsorbens kann weitgehend frei und somit den individuellen Erfordernissen entsprechend eingestellt werden. Es hat sich bewährt, je 100 Gew.-Teile flüssiger Phase 0,05 bis 30, vorzugsweise 0,1 bis 5 und insbesondere 0,1 bis 1 Gew.-Teile Adsorbens einzusetzen. Nach beendeter Umsetzung kann das Adsorbens aus dem Prozess abgetrennt und in das Veresterungsgefäß zurückgeführt und wieder eingesetzt werden. Der Wiedereinsatz ist sooft möglich, bis die Entfärbekraft des Adsorbens erschöpft ist. Es ist aber auch möglich, das Adsorbens in dem Rohprodukt zu belassen und während des Aufarbeitungsverfahrens an einer beliebigen aber zweckmäßigen Stufe abzutrennen.

Es ist ebenfalls möglich, während der Veresterungsreaktion ein inertes Gas, beispielsweise Stickstoff, Kohlendioxid oder die Edelgase, durch den Reaktionsansatz zu leiten, um das Reaktionswasser auszutreiben.

Nach Abtrennung der überschüssigen aliphatischen Monocarbonsäure als Zwischenfraktion wird der erhaltene Rohester einer Behandlung mit Wasserdampf unterzogen, die beispielsweise in einfacher Form durch Einleiten von Wasserdampf in das Rohprodukt erfolgen kann. Ein Vorteil der Wasserdampfbehandlung ist, dass in ihrem Verlauf noch vorhandener Katalysator zerstört und in gut abfiltrierbare Hydrolyseproduke überführt wird. Wird die Veresterungsreaktion in Gegenwart eines Adsorbens durchgeführt, erleichtert das bereits anwesende Adsorbens die Abscheidung der Katalysatorfolgeprodukte. Ansonsten kann es sich als vorteilhaft erweisen, das Adsorbens zu Beginn der Wasserdampfbehandlung zuzusetzen. Die Anwesenheit eines Adsorbens während der Wasserdampfbehandlung wirkt sich ebenfalls vorteilhaft auf die Farbe und auf die Farbstabilität des Polyolesters aus. Es ist aber auch möglich, nach beendeter Veresterungsreaktion und Abtrennung überschüssiger Ausgangsverbindungen, also vor Durchführung der Wasserdampfdestillation, das Adsorbens abzufiltrieren.

Die Wasserdampfbehandlung wird im Allgemeinen bei Normaldruck durchgeführt, obwohl die Anwendung eines leichten Unterdrucks zweckmäßigerweise bis 400 hPa nicht ausgeschlossen ist. Die Wasserdampfbehandlung erfolgt im Allgemeinen bei Temperaturen von 100 bis 250°C, vorzugsweise von 150 bis 220°C und insbesondere von 170 bis 200°C und richtet sich auch nach den physikalischen Eigenschaften der jeweils herzustellenden Polyolester.

Bei dem Prozessschritt der Wasserdampfbehandlung erweist es sich als zweckmäßig, während der Aufheizperiode bis zum Erreichen der Arbeitstemperatur möglichst schonend vorzugehen, um den Rohester auf die erforderliche Temperatur für die Wasserdampfbehandlung zu erhitzen.

Die Dauer der Wasserdampfbehandlung lässt sich durch Routineversuche ermitteln und sie wird im Allgemeinen über einen Zeitraum von 0,5 bis 5 Stunden durchgeführt. Eine zu lange Wasserdampfbehandlung führt zu einer ungewünschten Erhöhung der Farbzahl des Polyolesters und ist daher zu vermeiden. Auch beobachtet man eine verstärkte Abbaureaktion des Polyolesters zu sauer reagierenden Verbindungen, deren Gehalt sich in einem Anstieg der Neutralisationszahl oder Säurezahl beispielsweise bestimmt nach DIN EN ISO 3682/ASTM D 1613, zeigt. Des Weiteren kann bei Polyolestern auf Basis von Etherdiolen, wie zum Beispiel Triethylenglykol oder Tetraethylenglykol, ein unerwünschter Abbau der Etherkette einsetzen. Bei einer zu kurzen Behandlungsdauer ist die Restsäure- und Wasserentfernung nicht ausreichend wirksam und der gewünschte Polyolester weist noch eine zu hohe unerwünschte Säurezahl und einen zu hohen Wassergehalt auf. Auch wird bei zu kurzer Behandlungsdauer nur ein geringer vorteilhafter Effekt auf die Farbzahl des Polyolesters beobachtet.

Die bei der Wasserdampfdestillation abgetrennten Reste an aliphatischer Monocarbonsäure, die man auch als dritten Schnitt oder als Nachlauffraktion ansehen kann, sind wiederum mit farbgebenden Komponenten belastet und werden aus dem Prozess ausgeschleust. Diese Fraktion kann getrennt von der oder zusammen mit der im ersten Schnitt oder als Vorlauffraktion abgetrennten aliphatischen Monocarbosäure in einer separaten Destillationsstufe aufgereinigt und wieder in den Veresterungsprozess zurückgeführt werden. An die Wasserdampfbehandlung schließt sich gegebenenfalls nach Filtration des Adsorbens und weiterer angefallener Feststoffe die Trocknung des Polyolesters an, beispielsweise in dem man ein inertes Gas durch das Produkt bei erhöhter Temperatur leitet. Es kann auch bei erhöhter Temperatur gleichzeitig ein Unterdruck angelegt werden und gegebenenfalls ein inertes Gas durch das Produkt geleitet werden. Auch ohne Einwirken eines inerten Gases kann nur bei erhöhter Temperatur oder nur bei geringerem Druck gearbeitet werden. Die jeweiligen Trocknungsbedingungen, wie Temperatur, Druck und Dauer lassen sich durch einfache Vorversuche ermitteln. Im Allgemeinen arbeitet man bei Temperaturen im Bereich von 80 bis 250°C, vorzugsweise 100 bis 180°C und bei Drücken von 0,2 bis 500 hPa, vorzugsweise 1 bis 200 hPa und insbesondere 1 bis 20 hPa. Darauf wird der Rohester, falls noch nicht erfolgt, filtriert, um ihn von den Feststoffen, den Hydrolyseprodukten des Katalysators und dem Adsorbens, falls in der Veresterungsstufe oder vor der Wasserdampfbehandlung zugesetzt, zu befreien. Die Filtration erfolgt in konventionellen Filtrierapparaturen bei normaler Temperatur oder bei Temperaturen bis 120°C. Die Filtration kann durch gängige Filtrierhilfsmittel wie Cellulose, Kieselgel, Kieselgur, Holzmehl, unterstützt werden. Ihr Einsatz ist jedoch auf Ausnahmefälle beschränkt.

Durch die Maßnahme der fraktionierten Abtrennung der nicht umgesetzten und überschüssigen aliphatischen Monocarbonsäure können die farbgebenden Komponenten auf einfache Weise aus dem Prozess entfernt werden, so dass die Menge an Adsorbentien, die in einigen Fällen zur Farbaufhellung zugesetzt wird, vermindert werden kann.

Nach der Wasserdampfbehandlung oder nach der Trocknung kann auch eine weitere Behandlung des Polyolesters mit Oxidationsmitteln, beispielsweise mit einer wässrigen Wasserstoffperoxidlösung oder mit Ozon oder ozonhaltigen Gasen, anstelle einer Behandlung mit Adsorbentien, zum Beispiel mit Aktivkohle, vorgesehen werden, um die Farbzahl zu verbessern. Diese Maßnahme ist aber nur auf wenige Sonderfälle beschränkt. Die Verwendung einer wässrigen Wasserstoffperoxidlösung oder von Ozon oder ozonhaltigen Gasen hat gegenüber dem Einsatz von festen Adsorbentien jedoch den Vorteil, dass zusätzliche Filtrationsschritte entbehrlich werden.

Die als Zwischenfraktion angefallene und nur wenig belastete aliphatische Monocarbonsäure kann häufiger wiedereingesetzt werden, ohne dass es zu Minderungen in der Farbqualität des gewünschten Polyols kommt. Werden hingegen sämtliche erhaltenen Fraktionen an aliphatischer Monocarbonsäure vereinigt und wieder in den Prozess zurückgeführt, ist nur ein begrenzter Wiedereinsatz aufgrund der ansteigenden Farbzahl des Polyolesters möglich. Werden daher gemäß der erfindungsgemäßen Arbeitsweise die farbbelasteten Säurefraktionen während der Veresterungsreaktion und während der Aufarbeitung abgetrennt, lässt sich die als zweiter Schnitt oder als Zwischenfraktion zurückgewonnene aliphatische Monocarbonsäure ergiebiger ausnutzen.

Um in der Veresterungsreaktion den gewünschten Säureüberschuss bezogen auf Polyol einzustellen, können die Zwischenfraktionen gesammelt werden und in den Veresterungsprozess, gegebenenfalls nach weiterer Frischsäurezugabe wieder zurückgeführt oder als Einsatzmaterial für Folgeansätze wieder verwendet werden.

Es werden hellfarbige Polyolester erhalten, die auch den übrigen Spezifikationen, wie Wassergehalt, Restsäuregehalt, Restgehalt an Katalysatorbestandteilen und Restgehalt an Monoester genügen.

Die als Ausgangsstoffe für das erfindungsgemäße Verfahren eingesetzten mehrwertigen Alkohole oder Polyole genügen der allgemeinen Formel (I)

R(OH)ₙ (I)

in der R einen aliphatischen oder cycloaliphatischen Kohlenwasserstoffrest mit 2 bis 20, vorzugsweise 2 bis 10 Kohlenstoffatomen und n eine ganze Zahl von 2 bis 8, vorzugsweise 2, 3, 4, 5 oder 6 bedeuten.

Als Polyole geeignet sind ebenfalls Verbindungen der allgemeinen Formel (II)

H-(-O-[-CR¹R²-]ₘ-)ₒ-OH (II)

in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten.

Als Polyole, die nach dem erfindungsgemäßen Verfahren zu hellfarbigen Polyolestern umgesetzt werden können, eignen sich beispielsweise 1,3-Propandiol, 1,3-Butandiol, 1,4-Butandiol, Neopentylglykol, 2,2-Dimethylolbutan, Trimethylolethan, Trimethylolpropan, Di-Trimethylolpropan, Trimethylolbutan, 2,2,4-Trimethylpentan-1,3-diol, 1,2- Hexandiol, 1,6-Hexandiol, Pentaerythrit oder Di-Pentaerythrit oder 3(4),8(9)-Dihydroxymethyltricyclo[5.2.1,0^{2.6}]decan.

Als weitere Polyole kommen Ethylenglykol und 1,2-Propylenglykol und deren Oligomeren, insbesondere Di-, Tri- und Tetraethylenglykol oder Dipropylenglykol, Tripropylenglykol oder Tetrapropylenglykol in Betracht. Ethylen- und Propylenglykole sind industriell produzierte Chemikalien. Basissubstanz zu ihrer Herstellung ist Ethylenoxid und Propylenoxid, aus dem man 1,2-Ethylenglykol und 1,2-Propylenglykol durch Erhitzen mit Wasser unter Druck gewinnt. Diethylenglykol erhält man durch Ethoxylierung aus Ethylenglykol. Triethylenglykol fällt, wie Tetraethylenglykol, als Nebenprodukt bei der Hydrolyse von Ethylenoxid zur Herstellung von Ethylenglykol an. Beide Verbindungen können auch durch Umsetzung von Ethylenglykol mit Ethylenoxid synthetisiert werden. Dipropylenglykol, Tripropylenglykol, Tetrapropylenglykol und höhere Propoxylierungsprodukte sind aus der mehrfachen Addition von Propylenoxid an 1,2-Propylenglykol zugänglich.

Zur Gewinnung von hellfarbigen Polyolestern nach dem erfindungsgemäßen Prozess setzt man lineare oder verzweigte, aliphatische Monocarbonsäuren mit 3 bis 20 Kohlenstoffatomen im Molekül ein. Obgleich man gesättigten Säuren in vielen Fällen den Vorzug gibt, können, in Abhängigkeit vom jeweiligen Anwendungsgebiet der Weichmacher oder Schmiermittel, auch ungesättigte Carbonsäuren als Reaktionskomponente zur Estersynthese verwendet werden. Beispiele für Monocarbonsäuren als Bausteine von Polyolestern sind Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methylhexansäure, Cyclohexancarbonsäure, 2-Ethylhexansäure, n-Nonansäure, 2-Methyloctansäure, Isononansäure, 3,5,5-Trimethylhexansäure, 2-Propylheptansäure, 2-Methylundecansäure, Isoundecancarbonsäure, Tricyclodecancarbonsäure und Isotridecancarbonsäure. Besonders bewährt hat sich das neue Verfahren für die Herstellung von Polyolestern des Monoethylenglykols bzw. der oligomeren Ethylenglykole sowie des 1,2-Propylenglykols bzw. der oligomeren Propylenglykole mit C₄- bis C₁₃- bzw. C₅- bis C₁₀-Monocarbonsäuren sowie zur Herstellung von Polyolestern auf Basis von 1,3-Butandiol, Neopentylglykol, 2,2,4-Trimethylpentan-1,3-diol, Trimethylolpropan, Di-Trimethylolpropan, Pentaerythrit oder 3(4),8(9)-Dihydroxymethyltricyclo[5.2.1.0^{2.6}]decan.

Die Polyolester des Ethylenglykols sowie seine Oligomeren eignen sich hervorragend als Weichmacher für alle gängigen hochpolymeren thermoplastischen Stoffe. Besonders bewährt haben sie sich als Zusatz zu Polyvinylbutyral, das mit Glykolestern versetzt als Zwischenschicht zur Herstellung von Mehrschichten- oder Verbundgläsern verwendet wird. Sie können ebenfalls als Koaleszenzmittel oder Filmbildehilfsmittel in wässrigen Dispersionen von Kunststoffen, die als Beschichtungsmittel vielfältige Anwendung finden, eingesetzt werden. Nach dem erfindungsgemäßen Herstellungsverfahren lassen sich auf einfache Weise Polyolester mit ausgezeichneten Farbeigenschaften herstellen, die auch weiteren Qualitätsansprüchen, wie geringem Geruch oder einer geringen Säurezahl genügen. Besonders eignet sich das erfindungsgemäße Verfahren zur Herstellung von Triethylenglykol-di-2-ethylhexanoat (3G8 Ester), Tetraethylenglykol-di-n-heptanoat (4G7 Ester), Triethylenglykol-di-2-ethylbutyrat (3G6 Ester), Triethylenglykol-di-n-heptanoat (3G7 Ester) oder Tetraethylenglykol-di-2-ethylhexanoat (4G8 Ester).

Das erfindungsgemäße Verfahren kann kontinuierlich oder absatzweise in den für die chemische Technik typischen Reaktionsapparaten durchgeführt werden. Bewährt haben sich Rührkessel oder Reaktionsrohre, wobei die absatzweise Reaktionsführung die bevorzugte ist.

In den folgenden Beispielen wird das erfindungsgemäße Verfahren näher erläutert, es ist jedoch nicht auf die beschriebene Ausführungsform beschränkt.

### Ausführungsbeispiele:

### Beispiel 1:

Herstellung von Triethylenglykol-di-2-ethylhexanoat (3G8 Ester); Veresterung in Gegenwart von Aktivkohle mit Frischsäure

Die Veresterung von Triethylenglykol mit 2-Ethylhexansäure wurde in einem beheizbaren 1 l - Vierhalskolben durchgeführt, versehen mit Rührer, Innenthermometer und einem Wasserabscheider.

In dem Kolben wurden 250 Gramm (1,66 mol) Triethylenglykol und 680 Gramm (4,72 mol) frische 2-Ethylhexansäure sowie 0,4 Gew.-% Aktivkohle, bezogen auf den gesamten Reaktionsansatz, vorgelegt. Unter Rühren und unter Anlegen eines leichten Unterdrucks von 900 hPa wurde der Ansatz auf 225°C erhitzt. Bei Erreichen dieser Temperatur wurde der Druck schrittweise auf 400 hPa reduziert und gebildetes Reaktionswasser an dem Wasserabscheider abgenommen, während 2-Ethylhexansäure in das Reaktionsgefäß zurückfloss. Der Reaktionsverlauf wurde durch kontinuierliche Auswaage des über den Wasserabscheider ausgeschleusten Wassers sowie durch den Verlauf der Hydroxylzahl verfolgt. Nach insgesamt 14,5 Stunden Reaktionszeit wurde bei einer Resthydroxylzahl von 4,2 mg KOH/g (nach DIN 53240) die Reaktion beendet.

Im Wasserabscheider fielen nach Beendigung der Veresterungsreaktion 13,3 g 2-Ethylhexansäure als erster Schnitt oder als Vorfraktion an. Diese Fraktion wurde für den nächsten Veresterungsansatz nicht mehr eingesetzt.

Anschließend wurde über einen Zeitraum von 3,75 Stunden bei einer Temperatur von 200°C und bei einem Druck von 20 hPa die überschüssige 2-Ethylhexansäure abdestilliert. Man erhielt 187,1 g 2-Ethylhexansäure als Zwischenfraktion, die für nachfolgende Veresterungsansätze wieder verwendet werden konnte.

Es folgte eine Wasserdampfdestillation über einen Zeitraum von 2,5 Stunden bei 200°C und bei Normaldruck. Neben der angefallenen Wassermenge wurde als dritte Fraktion noch eine Restmenge an 2-Ethylhexansäure von 0,7 g zurückerhalten, die ebenfalls nicht mehr für nachfolgende Veresterungsansätze eingesetzt wurde.

Nach abschließender Filtration von der Aktivkohle erhielt man hellfarbiges Triethylenglykol-di-2-ethylhexanoat mit der in der Tabelle 1 angegebenen Farbzahl.

### Beispiel 2:

Herstellung von Triethylenglykol-di-2-ethylhexanoat (3G8 Ester); Veresterung in Gegenwart von Aktivkohle, Wiedereinsatz der Zwischenfraktion aus vorhergehenden Veresterungsansätzen

Beispiel 2 wurde analog Beispiel 1 durchgeführt mit der einzigen Ausnahme, dass anstelle von frischer 2-Ethylhexansäure die aus vorhergehenden Veresterungsansätzen gesammelte 2-Ethylhexansäure-Zwischenfraktion eingesetzt wurde.

### Beispiel 3 (Vergleichsbeispiel):

Herstellung von Triethylenglykol-di-2-ethylhexanoat (3G8 Ester); Veresterung in Gegenwart von Aktivkohle, Wiedereinsatz der gesamten Rücksäure aus vorhergehenden Veresterungsansätzen

Beispiel 3 wurde analog Beispiel 2 durchgeführt mit der einzigen Ausnahme, dass anstelle der 2-Ethylhexansäure-Zwischenfraktion die gesamte aus den vorhergehenden Veresterungsansätzen zurückgewonnene 2-Ethylhexansäure eingesetzt wurde.

Die nach den Beispielen 1 bis 3 erhaltenen Farbzahlen der aufgearbeiteten Triethylenglykol-di-2-ethylhexanoat-Ester sind in der nachfolgenden Tabelle 1 aufgeführt. Die gaschromatographisch ermittelten Estergehalte sowie die übrigen Kennzahlen, wie Säurerestgehalt oder Wassergehalt, stimmten überein.

**Tabelle 1: Farbzahlen von Triethylenglykol-di-2-ethylhexanoat, hergestellt nach den Beispielen 1, 2 und 3**

| | Beispiel 1 | Beispiel 2 | Beispiel 3 |
|---|---|---|---|
| Farbzahl nach Hazen (DIN ISO 6271) | 24 | 15 | 45 |

Durch die erfindungsgemäße Maßnahme, die bei der Veresterung zurückgewonnene aliphatische Monocarbonsäure partiell in die Veresterungsstufe zurückzuführen oder partiell für nachfolgende Veresterungsansätze einzusetzen, lassen sich Polyolester mit hervorragender Farbzahl erhalten, die ihren Einsatz in einer Vielzahl von Anwendungen ermöglicht.

## Patentansprüche

1. Verfahren zur Herstellung von Polyolestern durch Umsetzung von Polyolen mit linearen oder verzweigten aliphatischen Monocarbonsäuren mit 3 bis 20 Kohlenstoffatomen im Überschuss und anschließende Aufarbeitung des Reaktionsgemisches mittels Wasserdampfbehandlung, **dadurch gekennzeichnet, dass** man in einem ersten Schnitt die während der Umsetzung abgetrennte aliphatische Monocarbonsäure vollständig oder teilweise aus dem Prozess entfernt, in einem zweiten Schnitt die nach beendeter Umsetzung im Reaktionsgemisch noch vorhandene aliphatische Monocarbonsäure abtrennt und wieder vollständig in die Veresterungsreaktion zurückführt und in einem dritten Schnitt die bei der Wasserdampfbehandlung des Umsetzungsproduktes abgetrennte Restmenge an aliphatischer Monocarbonsäure vollständig aus dem Prozess entfernt, mit der Maßgabe, dass die aliphatische Monocarbonsäure einen niedrigeren Siedepunkt als das eingesetzte Polyol aufweist und sich an die Wasserdampfbehandlung die Trocknung des Polyolesters anschließt.

2. Verfahren gemäß Anspruch 1, **dadurch gekennzeichnet, dass** man bei der Umsetzung der Ausgangsverbindungen auf eine Temperatur bis auf maximal 280°C, vorzugsweise bis auf 250°C, erhitzt und bei konstant gehaltener Temperatur den Druck von Stufe zu Stufe erniedrigt.

3. Verfahren gemäß Anspruch 1, **dadurch gekennzeichnet, dass** man bei der Umsetzung der Ausgangsverbindungen bei konstant eingestelltem Druck von Stufe zu Stufe bis auf eine maximale Temperatur von 280°C erhitzt.

4. Verfahren gemäß Anspruch 1, **dadurch gekennzeichnet, dass** man bei der Umsetzung der Ausgangsverbindungen bei von Stufe zu Stufe ansteigender Temperatur auf maximal 280°C erhitzt und auch den Druck von Stufe zu Stufe erniedrigt.

5. Verfahren gemäß Anspruch 4, **dadurch gekennzeichnet, dass** man bei der Umsetzung der Ausgangsverbindungen in einer ersten Stufe bei einer Temperatur bis auf 190°C und bei einem Druck bis auf 600 hPa reagieren lässt und die Reaktion in einer zweiten Stufe durch Erhöhung der Temperatur bis auf 250°C und bei einem Druck bis auf 300 hPa zu Ende führt.

6. Verfahren gemäß einem oder mehreren der Ansprüche 1 bis 5, **dadurch gekennzeichnet, dass** man die Umsetzung der Ausgangsverbindungen in Gegenwart eines Katalysators durchführt.

7. Verfahren gemäß Anspruch 6, **dadurch gekennzeichnet, dass** man als Katalysator Titan, Zirkonium oder Zinn enthaltende Katalysatoren verwendet.

8. Verfahren gemäß einem oder mehreren der Ansprüche 1 bis 7, **dadurch gekennzeichnet, dass** man die Umsetzung der Ausgangsverbindungen in Gegenwart eines Adsorbens durchführt.

9. Verfahren gemäß Anspruch 8, **dadurch gekennzeichnet, dass** das Adsorbens in einer Menge von 0,05 bis 30, vorzugsweise von 0,1 bis 5,0 und insbesondere von 0,1 bis 1,0 Gew.-Teilen je 100 Gew.-Teilen flüssiger Phase eingesetzt wird.

10. Verfahren gemäß Anspruch 8 oder 9, **dadurch gekennzeichnet, dass** man als Adsorbens Silicagel, Kieselgel, Kieselguhr, Aluminiumoxid, Aluminiumoxidhydrate, Tone, Carbonate oder Aktivkohle verwendet.

11. Verfahren gemäß einem oder mehreren der Ansprüche 1 bis 10, **dadurch gekennzeichnet, dass** man die Umsetzung der Ausgangsverbindungen in Gegenwart eines Inertgases durchführt.

12. Verfahren gemäß einem oder mehreren der Ansprüche 1 bis 11, **dadurch gekennzeichnet, dass** man die Wasserdampfbehandlung bei einer Temperatur von 100 bis 250°C, vorzugsweise von 150 bis 220°C und insbesondere von 170 bis 200°C durchführt.

13. Verfahren gemäß einem oder mehreren der Ansprüche 1 bis 12, **dadurch gekennzeichnet, dass** der Polyolester nach der Wasserdampfbehandlung bei Temperaturen von 80 bis 250°C, vorzugsweise 100 bis 180°C und bei Drücken von 0,2 bis 500 hPa, vorzugsweise 1 bis 200 hPa und insbesondere von 1 bis 20 hPa getrocknet wird.

14. Verfahren gemäß Anspruch 13, **dadurch gekennzeichnet, dass** der Polyolester in Gegenwart eines Inertgases getrocknet wird.

15. Verfahren gemäß einem oder mehreren der Ansprüche 1 bis 14, **dadurch gekennzeichnet, dass** der Polyolester nach der Wasserdampfbehandlung filtriert wird.

16. Verfahren gemäß Anspruch 13 oder 14, **dadurch gekennzeichnet, dass** der Polyolester nach der Trocknung filtriert wird.

17. Verfahren gemäß einem oder mehreren der Ansprüche 12 bis 16, **dadurch gekennzeichnet, dass** der Polyolester nach der Wasserdampfbehandlung oder nach der Trocknung mit einem Oxidationsmittel behandelt wird.

18. Verfahren gemäß Anspruch 17, **dadurch gekennzeichnet, dass** man als Oxidationsmittel Wasserstoffperoxid, Ozon oder ozonhaltige Gase verwendet.

19. Verfahren gemäß einem oder mehreren der Ansprüche 1 bis 18, **dadurch gekennzeichnet, dass** man als Polyole Verbindungen der allgemeinen Formel (I)
R(OH)ₙ (I)
verwendet, in der R einen aliphatischen oder cycloaliphatischen Kohlenwasserstoffrest mit 2 bis 20, vorzugsweise 2 bis 10 Kohlenstoffatomen und n eine ganze Zahl von 2 bis 8, vorzugsweise 2, 3, 4, 5 oder 6, bedeuten.

20. Verfahren gemäß einem oder mehreren der Ansprüche 1 bis 18, **dadurch gekennzeichnet, dass** man als Polyole Verbindungen der allgemeinen Formel (II)
H-(-O-[-CR¹R²-]ₘ-)ₒ-OH (II)
verwendet, in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten.

21. Verfahren gemäß Anspruch 19, **dadurch gekennzeichnet, dass** man als Polyole 1,2-Propandiol, 1,3-Propandiol, 1,3-Butandiol, 1,4-Butandiol, Neopentylglykol, 2,2-Dimethylolbutan, Trimethylolethan, Trimethylolpropan, Trimethylolbutan, 2,2,4-Trimethylpentan-1,3-diol, 1,2-Hexandiol, 1,6-Hexandiol, Pentaerythrit, Ethylenglykol oder 3(4),8(9)-Dihydroxymethyltricyclo[5.2.1.0^{2.6}]decan verwendet.

22. Verfahren gemäß Anspruch 20, **dadurch gekennzeichnet, dass** man als Polyole Di-Trimethylolpropan, Di-Pentaerythrit, Diethylenglykol, Triethylenglykol, Tetraethylenglykol, Dipropylenglykol, Tripropylenglykol oder Tetrapropylenglykol verwendet.

23. Verfahren gemäß einem oder mehreren der Ansprüche 1 bis 22, **dadurch gekennzeichnet, dass** man als aliphatische Monocarbonsäure Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methylhexansäure, 2-Ethylhexansäure, n-Nonansäure, 2-Methyloctansäure, Isononansäure, 3,5,5-Trimethylhexansäure oder 2-Propylheptansäure umsetzt.

24. Verfahren gemäß einem oder mehreren der Ansprüche 1 bis 23 zur Herstellung von Triethylenglykol-di-2-ethylhexanoat, Tetraethylenglykol-di-n-heptanoat, Triethylenglykol-di-2-ethylbutyrat, Triethylenglykol-di-n-heptanoat oder Tetraethylenglykol-di-2-ethylhexanoat.

## Claims

1. Process for preparing polyol esters by reacting polyols with linear or branched aliphatic monocarboxylic acids having 3 to 20 carbon atoms in excess and then working up the reaction mixture by means of steam treatment, **characterized in that** in a first fraction the aliphatic monocarboxylic acid removed during the reaction is removed fully or partly from the process, in a second fraction the aliphatic monocarboxylic acid still present in the reaction mixture after reaction has ended is removed and recycled fully back into the esterification reaction, and in a third fraction the residual amount of aliphatic monocarboxylic acid removed in the course of steam treatment of the reaction product is removed fully from the process, with the proviso that the aliphatic monocarboxylic acid has a lower boiling point than the polyol used and the steam treatment is followed by the drying of the polyol ester.

2. Process according to Claim 1, **characterized in that** the reaction of the starting compounds involves heating to a temperature up to a maximum of 280°C, preferably up to 250°C, and lowering the pressure from stage to stage with the temperature kept constant.

3. Process according to Claim 1, **characterized in that** the reaction of the starting compounds involves heating at constant pressure from stage to stage up to a maximum temperature of 280°C.

4. Process according to Claim 1, **characterized in that** the reaction of the starting compounds involves heating at a temperature rising from stage to stage to a maximum of 280°C, and also lowering the pressure from stage to stage.

5. Process according to Claim 4, **characterized in that** the reaction of the starting compounds involves allowing them to react in a first stage at a temperature up to 190°C and at a pressure up to 600 hPa, and conducting the reaction to completion in a second stage by increasing the temperature up to 250°C and at a pressure up to 300 hPa.

6. Process according to one or more of Claims 1 to 5, **characterized in that** the reaction of the starting compounds is performed in the presence of a catalyst.

7. Process according to Claim 6, **characterized in that** the catalysts used are catalysts comprising titanium, zirconium or tin.

8. Process according to one of more of Claims 1 to 7, **characterized in that** the reaction of the starting compounds is performed in the presence of an adsorbent.

9. Process according to Claim 8, **characterized in that** the adsorbent is used in an amount of 0.05 to 30 parts, preferably of 0.1 to 5.0 parts and especially of 0.1 to 1.0 part by weight per 100 parts by weight of liquid phase.

10. Process according to Claim 8 or 9, **characterized in that** the adsorbent used is silica gel, kieselguhr, aluminum oxide, aluminum oxide hydrates, clays, carbonates or activated carbon.

11. Process according to one or more of Claims 1 to 10, **characterized in that** the reaction of the starting compounds is performed in the presence of an inert gas.

12. Process according to one or more of Claims 1 to 11, **characterized in that** the steam treatment is performed at a temperature of 100 to 250°C, preferably of 150 to 220°C and especially of 170 to 200°C.

13. Process according to one or more of Claims 1 to 12, **characterized in that** the polyol ester, after the steam treatment, is dried at temperatures of 80 to 250°C, preferably 100 to 180°C, and at pressures of 0.2 to 500 hPa, preferably 1 to 200 hPa and especially of 1 to 20 hPa.

14. Process according to Claim 13, **characterized in that** the polyol ester is dried in the presence of an inert gas.

15. Process according to one or more of Claims 1 to 14, **characterized in that** the polyol ester is filtered after the steam treatment.

16. Process according to Claim 13 or 14, **characterized in that** the polyol ester is filtered after the drying.

17. Process according to one or more of Claims 12 to 16, **characterized in that** the polyol ester is treated with an oxidizing agent after the steam treatment or after the drying.

18. Process according to Claim 17, **characterized in that** the oxidizing agents used are hydrogen peroxide, ozone or ozone-containing gases.

19. Process according to one or more of Claims 1 to 18, **characterized in that** the polyols used are compounds of the general formula (I)
R(OH)ₙ (I)
in which R is an aliphatic or cycloaliphatic hydrocarbon radical having 2 to 20 and preferably 2 to 10 carbon atoms, and n is an integer of 2 to 8, preferably 2, 3, 4, 5 or 6.

20. Process according to one or more of Claims 1 to 18, **characterized in that** the polyols used are compounds of the general formula (II)
H-(-O-[-CR¹R²-]ₘ-)ₒ-OH (II)
in which R¹ and R² are each independently hydrogen, an alkyl radical having 1 to 5 carbon atoms, preferably methyl, ethyl or propyl, or a hydroxyalkyl radical having 1 to 5 carbon atoms, preferably the hydroxymethyl radical, m is an integer of 1 to 10, preferably 1 to 8 and especially 1, 2, 3 or 4, o is an integer of 2 to 15, preferably 2 to 8 and especially 2, 3, 4 or 5.

21. Process according to Claim 19, **characterized in that** the polyols used are 1,2-propanediol, 1,3-propanediol, 1,3-butanediol, 1,4-butanediol, neopentyl glycol, 2,2-dimethylolbutane, trimethylolethane, trimethylolpropane, trimethylolbutane, 2,2,4-trimethylpentane-1,3-diol, 1,2-hexanediol, 1,6-hexanediol, pentaerythritol, ethylene glycol or 3(4),8(9)-dihydroxymethyltricyclo[5.2.1.0^{2.6}]decane.

22. Process according to Claim 20, **characterized in that** the polyols used are ditrimethylolpropane, dipentaerythritol, diethylene glycol, triethylene glycol, tetraethylene glycol, dipropylene glycol, tripropylene glycol or tetrapropylene glycol.

23. Process according to one or more of Claims 1 to 22, **characterized in that** the aliphatic monocarboxylic acid converted is propionic acid, n-butyric acid, isobutyric acid, n-pentanoic acid, 2-methylbutyric acid, 3-methylbutyric acid, 2-methylpentanoic acid, n-hexanoic acid, 2-ethylbutyric acid, n-heptanoic acid, 2-methylhexanoic acid, 2-ethylhexanoic acid, n-nonanoic acid, 2-methyloctanoic acid, isononanoic acid, 3,5,5-trimethylhexanoic acid or 2-propylheptanoic acid.

24. Process according to one or more of Claims 1 to 23 for preparing triethylene glycol di-2-ethylhexanoate, tetraethylene glycol di-n-heptanoate, triethylene glycol di-2-ethylbutyrate, triethylene glycol di-n-heptanoate or tetraethylene glycol di-2-ethylhexanoate.

## Revendications

1. Procédé pour la préparation de polyolesters par transformation de polyols avec des acides monocarboxyliques aliphatiques, linéaires ou ramifiés, comprenant 3 à 20 atomes de carbone en excès et traitement consécutif du mélange réactionnel par traitement à la vapeur d'eau, **caractérisé en ce que**, dans une première fraction, l'acide monocarboxylique aliphatique séparé pendant la transformation est éliminé complètement ou partiellement du procédé, dans une deuxième fraction, l'acide monocarboxylique aliphatique encore présent dans le mélange réactionnel après la transformation est séparé et à nouveau recyclé complètement dans la réaction d'estérification et, dans une troisième fraction, la quantité résiduelle d'acide monocarboxylique aliphatique séparé lors du traitement à la vapeur d'eau du produit de transformation est éliminé complètement du procédé, à condition que l'acide monocarboxylique aliphatique présente un point d'ébullition inférieur à celui du polyol utilisé et que le traitement à la vapeur d'eau soit suivi du séchage du polyolester.

2. Procédé selon la revendication 1, **caractérisé en ce qu'**on chauffe lors de la transformation des composés de départ à une température d'au maximum jusqu'à 280°C, de préférence jusqu'à 250°C, et on abaisse, en maintenant la température constante, la pression d'une étape à l'autre.

3. Procédé selon la revendication 1, **caractérisé en ce qu'**on chauffe lors de la transformation des composés de départ à une pression réglée à une valeur constante d'une étape à l'autre jusqu'à une température maximale de 280°C.

4. Procédé selon la revendication 1, **caractérisé en ce qu'**on chauffe lors de la transformation des composés de départ à une température augmentant d'une étape à l'autre jusqu'au maximum à 280°C et on abaisse également la pression d'une étape à l'autre.

5. Procédé selon la revendication 4, **caractérisé en ce qu'**on laisse réagir lors de la transformation des composés de départ, dans une première étape, à une température de jusqu'à 190°C et à une pression de jusqu'à 600 hPa et on conduit la réaction jusqu'à la fin dans une deuxième étape par augmentation de la température jusqu'à 250°C et à une pression jusqu'à 300 hPa.

6. Procédé selon l'une ou plusieurs des revendications 1 à 5, **caractérisé en ce qu'**on réalise la transformation des composés de départ en présence d'un catalyseur.

7. Procédé selon la revendication 6, **caractérisé en ce qu'**on utilise, comme catalyseur, des catalyseurs contenant du titane, du zirconium ou de l'étain.

8. Procédé selon l'une ou plusieurs des revendications 1 à 7, **caractérisé en ce qu'**on réalise la transformation des composés de départ en présence d'un adsorbant.

9. Procédé selon la revendication 8, **caractérisé en ce que** l'adsorbant est utilisé en une quantité de 0,05 à 30, de préférence de 0,1 à 5,0 et en particulier de 0,1 à 1,0 partie(s) en poids par 100 parties en poids de phase liquide.

10. Procédé selon la revendication 8 ou 9, **caractérisé en ce qu'**on utilise, comme adsorbant, le silicagel, le gel silicique, le kieselguhr, l'oxyde d'aluminium, l'oxyde d'aluminium hydraté, l'argile, des carbonates ou le charbon actif.

11. Procédé selon l'une ou plusieurs des revendications 1 à 10, **caractérisé en ce qu'**on réalise la transformation des composés de départ en présence d'un gaz inerte.

12. Procédé selon l'une ou plusieurs des revendications 1 à 11, **caractérisé en ce qu'**on réalise le traitement à la vapeur d'eau à une température de 100 à 250°C, de préférence de 150 à 220°C et en particulier de 170 à 200°C.

13. Procédé selon l'une ou plusieurs des revendications 1 à 12, **caractérisé en ce que** le polyolester est séché après le traitement à la vapeur d'eau à des températures de 80 à 250°C, de préférence de 100 à 180°C et à des pressions de 0,2 à 500 hPa, de préférence de 1 à 200 hPa et en particulier de 1 à 20 hPa.

14. Procédé selon la revendication 13, **caractérisé en ce que** le polyolester est séché en présence d'un gaz inerte.

15. Procédé selon l'une ou plusieurs des revendications 1 à 14, **caractérisé en ce que** le polyolester est filtré après le traitement à la vapeur d'eau.

16. Procédé selon la revendication 13 ou 14, **caractérisé en ce que** le polyolester est filtré après le séchage.

17. Procédé selon l'une ou plusieurs des revendications 12 à 16, **caractérisé en ce que** le polyolester est traité par un oxydant après le traitement à la vapeur d'eau ou après le séchage.

18. Procédé selon la revendication 17, **caractérisé en ce qu'**on utilise, comme oxydant, du peroxyde d'hydrogène, de l'ozone ou des gaz contenant de l'ozone.

19. Procédé selon l'une ou plusieurs des revendications 1 à 18, **caractérisé en ce qu'**on utilise, comme polyols, des composés de formule générale (I)
R(OH)ₙ (I)
dans laquelle R signifie un radical hydrocarboné aliphatique ou cycloaliphatique comprenant 2 à 20, de préférence 2 à 10 atomes de carbone et n vaut un nombre entier de 2 à 8, de préférence 2, 3, 4, 5 ou 6.

20. Procédé selon l'une ou plusieurs des revendications 1 à 18, **caractérisé en ce qu'**on utilise, comme polyols, des composés de formule générale (II)
H-(-O-[-CR¹R²-]ₘ-)ₒ-OH (II)
dans laquelle R¹ et R² signifient, indépendamment l'un de l'autre, hydrogène, un radical alkyle comprenant 1 à 5 atomes de carbone, de préférence méthyle, éthyle ou propyle, ou un radical hydroxyalkyle comprenant 1 à 5 atomes de carbone, de préférence le radical hydroxyméthyle, m vaut un nombre entier de 1 à 10, de préférence de 1 à 8 et en particulier 1, 2, 3 ou 4, o vaut un nombre entier de 2 à 15, de préférence de 2 à 8 et en particulier 2, 3, 4 ou 5.

21. Procédé selon la revendication 19, **caractérisé en ce qu'**on utilise, comme polyols, le 1,2-propanediol, le 1,3-propanediol, le 1,3-butanediol, le 1,4-butanediol, le néopentylglycol, le 2,2-diméthylolbutane, le triméthyloléthane, le triméthylolpropane, le triméthylolbutane, le 2,2,4-triméthylpentane-1,3-diol, le 1,2-hexanediol, le 1,6-hexanediol, le pentaérythritol, l'éthylèneglycol ou le 3(4),8(9)-dihydroxyméthyltricyclo[5,2,1,0^{2,6}]décane.

22. Procédé selon la revendication 20, **caractérisé en ce qu'**on utilise, comme polyols, le di-triméthylolpropane, le di-pentaérythritol, le diéthylèneglycol, le triéthylèneglycol, le tétraéthylèneglycol, le dipropylèneglycol, le tripropylèneglycol ou le tétrapropylèneglycol.

23. Procédé selon l'une ou plusieurs des revendications 1 à 22, **caractérisé en ce qu'**on utilise comme acide monocarboxylique aliphatique les acides propionique, n-butyrique, isobutyrique, n-pentanoïque, 2-méthylbutyrique, 3-méthylbutyrique, 2-méthylpentanoïque, n-hexanoïque, 2-éthylbutyrique, n-heptanoïque, 2-méthylhexanoïque, 2-éthylhexanoïque, n-nonanoïque, 2-méthyloctanoïque, isononanoïque, 3,5,5-triméthylhexanoïque ou 2-propylheptanoïque.

24. Procédé selon l'une ou plusieurs des revendications 1 à 23 pour la préparation de di-2-éthylhexanoate de triéthylèneglycol, de di-n-heptanoate de tétraéthylèneglycol, de di-2-éthylbutyrate de triéthylèneglycol, de di-n-heptanoate de triéthylèneglycol ou de di-2-éthylhexanoate de tétraéthylèneglycol.
